# EUROPEAN PATENT APPLICATION

(11) **EP 3 998 429 A1**
(43) Date of publication of application: **18.05.2022**
(21) Application number: 20836139.4
(22) Date of filing: 06.07.2020
(51) Int. Cl.: F24F 11/63, G16H 50/30, G06N 20/00, A61B 5/00, A61B 5/01, A61B 5/16

(54) **LEARNING DEVICE, ESTIMATION DEVICE, AND ENVIRONMENT ADJUSTMENT SYSTEM**

(30) Priority: 08.07.2019 JP 2019126816; 08.07.2019 JP 2019126815; 20.05.2020 JP 2020088163
(71) Applicant: Daikin Industries, Ltd., Osaka-shi, Osaka 530-8323 (JP)
(72) Inventor: NISHIKAWA, Yuki, Osaka 530-8323 (JP); HASHIMOTO, Satoshi, Osaka 530-8323 (JP); HORI, Shouta, Osaka 530-8323 (JP); UCHIYAMA, Akira, Osaka 565-0871 (JP); HIGASHINO, Teruo, Osaka 565-0871 (JP)
(74) Representative: Global IP Europe Patentanwaltskanzlei
(86) International application number: PCT/JP2020/026384
(87) International publication number: WO 2021/006240

(57) **Abstract**

A sleep state of a sleeping person is accurately inferred by taking into account the fact that the core body temperature affects sleep. A learning apparatus (30) learns a quality of sleep of a sleeping person. The learning apparatus (30) includes an acquisition unit (31), a learning unit (32), and a generation unit (33). The acquisition unit (31) acquires, as a state variable, a feature quantity related to a core body temperature of the sleeping person or a feature quantity related to a skin temperature of the sleeping person. The feature quantity related to the core body temperature is determined based on at least a first core body temperature at sleep onset of the sleeping person. The feature quantity related to the skin temperature is determined based on at least a first skin temperature at the sleep onset of the sleeping person. The learning unit (32) learns the state variable and the quality of sleep in association with each other. Based on a learning result of the learning unit (32), the generation unit (33) generates a learning model. The learning model receives, as an input, the feature quantity related to the core body temperature or the feature quantity related to the skin temperature of the sleeping person and outputs the quality of sleep.

## Description

### Technical Field

The present disclosure relates to a learning apparatus, an inference apparatus, and an environment adjustment system.

### Background Art

NPL 1 (<Paper> The Effects of Pre-Bedtime Changes in Body Temperature on Sleep Onset (General)) discloses that the core body temperature affects sleep.

### Summary of Invention

### Technical Problem

A sleep state of a sleeping person is accurately inferred by taking into account the fact that the core body temperature affects sleep.

### Solution to Problem

A learning apparatus of a first aspect learns a quality of sleep of a sleeping person. The learning apparatus includes an acquisition unit, a learning unit, and a generation unit. The acquisition unit acquires a state variable. The state variable is a feature quantity related to a core body temperature of the sleeping person or a feature quantity related to a skin temperature of the sleeping person. The feature quantity related to the core body temperature is determined based on at least a first core body temperature at sleep onset of the sleeping person. The feature quantity related to the skin temperature is determined based on at least a first skin temperature at the sleep onset of the sleeping person. The learning unit learns the state variable and the quality of sleep in association with each other. Based on a learning result of the learning unit, the generation unit generates a learning model. The learning model receives, as an input, at least the feature quantity related to the core body temperature or the feature quantity related to the skin temperature of the sleeping person, and outputs the quality of sleep.

Thus, the learning apparatus generates the learning model capable of accurately inferring, by using the core body temperature, the quality of sleep that is the sleep state of the sleeping person.

A learning apparatus of a second aspect is the learning apparatus of the first aspect, in which the feature quantity related to the core body temperature is determined based further on a second core body temperature acquired before the first core body temperature. The feature quantity related to the skin temperature is determined based further on a second skin temperature acquired before the first skin temperature.

Thus, a more accurate learning result can be obtained.

A learning apparatus of a third aspect is the learning apparatus of the first aspect or the second aspect, in which the acquisition unit further acquires a feature quantity related to a normal core body temperature of the sleeping person or a feature quantity related to a normal skin temperature of the sleeping person.

Thus, a more accurate learning result can be obtained.

A learning apparatus of a fourth aspect is the learning apparatus of any of the first aspect to the third aspect, in which the acquisition unit acquires, as the state variable, an ambient temperature of the sleeping person at the sleep onset, a fingertip blood flow rate of the sleeping person, or an RGB image of the sleeping person.

Thus, a more accurate learning result can be obtained.

A learning apparatus of a fifth aspect is the learning apparatus of any of the first aspect to the fourth aspect, in which the quality of sleep is determined based on at least any one of a period from lying down to the sleep onset of the sleeping person, a proportion of deep sleep of the sleeping person, the number of times of arousal during sleep of the sleeping person, a duration of arousal during sleep of the sleeping person, a questionnaire on the quality of sleep for the sleeping person, a questionnaire on daytime performance for the sleeping person, a hormone secretion of the sleeping person, and a hormone concentration of the sleeping person.

A learning apparatus of a sixth aspect is the learning apparatus according to any of the first aspect to the fifth aspect, in which the feature quantity related to the core body temperature is determined based on at least any one of an amount of change in, a largest value of, a smallest value of, a mode value of, an average value of, a difference between the largest value and the smallest value of, a largest or smallest slope of, an average slope of, and an index related to the change in the core body temperature of the sleeping person in a predetermined period.

A learning apparatus of a seventh aspect is the learning apparatus according to any of the first aspect to the sixth aspect, in which the feature quantity related to the skin temperature is determined based on at least any one of an amount of change in, a largest value of, a smallest value of, a mode value of, an average value of, a difference between the largest value and the smallest value of, a largest or smallest slope of, an average slope of, and an index related to the change in the skin temperature of the sleeping person in a predetermined period.

A learning apparatus of an eighth aspect is the learning apparatus of any of the first aspect to the seventh aspect, in which the acquisition unit further acquires, as the state variable, any of a humidity, an illuminance, a chromaticity, a scent, and an air flow around the sleeping person at the sleep onset of the sleeping person.

Thus, a more accurate learning result can be obtained.

A learning apparatus of a ninth aspect is the learning apparatus of any of the first aspect to the eighth aspect, in which the learning unit performs learning by using a plurality of pieces of training data. The training data includes the state variable and the quality of sleep.

An inference apparatus of a tenth aspect infers the quality of sleep by using the learning model generated based on a learning result of the learning apparatus according to any of the first aspect to the ninth aspect.

Thus, the inference apparatus can infer an accurate quality of sleep.

An inference apparatus of an eleventh aspect is the inference apparatus of the tenth aspect that further includes a core body temperature feature quantity inference unit. The core body temperature feature quantity inference unit infers the feature quantity related to the core body temperature of the sleeping person from the feature quantity related to the skin temperature.

Thus, the inference apparatus can reduce a load imposed on the sleeping person by measurement of the core body temperature.

An inference apparatus of a twelfth aspect is the inference apparatus of the tenth aspect, in which the core body temperature feature quantity inference unit infers the feature quantity related to the core body temperature of the sleeping person by receiving, as an input, the feature quantity related to the skin temperature of the sleeping person, based on a learning result of the learning model that has learned the feature quantity related to the core body temperature of the sleeping person in association with the feature quantity related to the skin temperature of the sleeping person. The feature quantity related to the skin temperature of the sleeping person is determined based on at least the first skin temperature at sleep onset of the sleeping person. The feature quantity related to the core body temperature of the sleeping person is determined based on at least the first core body temperature at the sleep onset of the sleeping person.

An environment adjustment system of a thirteenth aspect includes the inference apparatus of any of the tenth aspect to the twelfth aspect and an environment adjustment apparatus. The environment adjustment apparatus includes an actuator and a control unit. The actuator adjusts an environment around a sleeping person. Based on the quality of sleep of the sleeping person inferred by the inference apparatus, the control unit controls an operation of the actuator.

Thus, the environment adjustment system can improve the quality of sleep of the sleeping person by adjusting the environment around the sleeping person.

### Brief Description of Drawings

[Fig. 1] Fig. 1 is a diagram illustrating an example of a learning system and an inference system.
[Fig. 2] Fig. 2 is a diagram illustrating a configuration of the learning system.
[Fig. 3] Fig. 3 is a diagram illustrating configurations of the inference system and an environment adjustment system 3.

### Description of Embodiments

### <First Embodiment

### (1) Overview

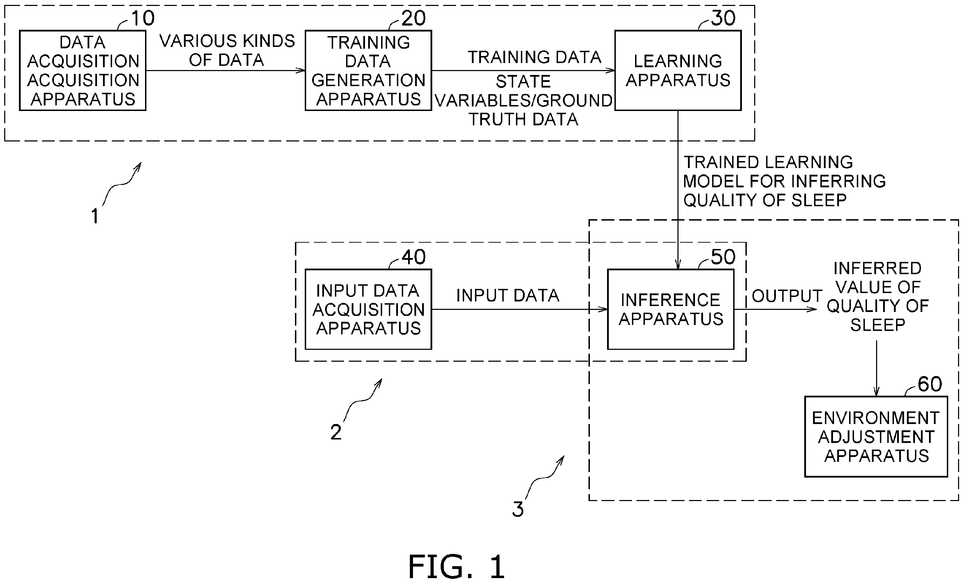

Fig. 1 is a diagram illustrating an example of a learning system 1, an inference system 2, and an environment adjustment system 3 according to a first embodiment. The learning system 1 acquires various kinds of data, performs learning based on the acquired data, and generates a learning model trained based on the learning result. The inference system 2 acquires various kinds of data, and infers the quality of sleep of a sleeping person by using the trained learning model generated by the learning system 1. Based on the quality of sleep of the sleeping person inferred by the inference system 2, the environment adjustment system 3 adjusts an environment around the sleeping person.

Details of the learning system 1, the inference system 2, and the environment adjustment system 3 will be described below.

### (2) Learning System 1

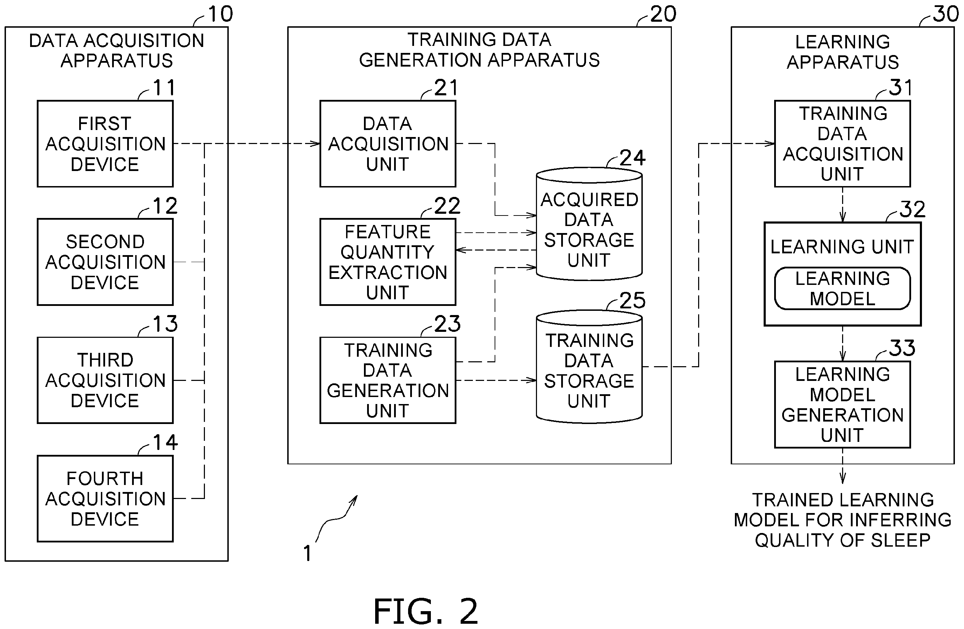

Fig. 2 is a schematic diagram illustrating an example of the learning system 1 according to the first embodiment. The learning system 1 mainly includes a data acquisition apparatus 10, a training data generation apparatus 20, and a learning apparatus 30. The individual apparatuses are capable of communicating with one another via a network such as Internet.

### (2-1) Data Acquisition Apparatus 10

The data acquisition apparatus 10 includes, for example, a first acquisition device 11 that acquires data of a temperature around a sleeping person, a second acquisition device 12 that acquires data of a skin temperature of the sleeping person, a third acquisition device 13 that acquires data of a core body temperature of the sleeping person, and a fourth acquisition device 14 that acquires data of a quality of sleep of the sleeping person. In the first embodiment, each of the first acquisition device 11, the second acquisition device 12, and the third acquisition device 13 continuously acquires the data from 30 minutes before the sleeping person lies down to when the sleeping person wakes up. However, the data may be acquired at predetermined time intervals or may be acquired at given timings. In the first embodiment, the fourth acquisition device 14 acquires the data after the sleeping person wakes up. Each of the pieces of data acquired by the first acquisition device 11, the second acquisition device 12, the third acquisition device 13, and the fourth acquisition device 14 is associated with information of the acquisition time of the piece of data.

Each of the pieces of data acquired by the first acquisition device 11, the second acquisition device 12, the third acquisition device 13, and the fourth acquisition device 14 is transmitted to the training data generation apparatus 20 via the network. Note that the first acquisition device 11, the second acquisition device 12, the third acquisition device 13, and the fourth acquisition device 14 may transmit, to the training data generation apparatus 20, only partial data corresponding to a request from the training data generation apparatus 20 among the acquired pieces of data.

### (2-1-1) First Acquisition Device 11

The first acquisition device 11 is a device such as a temperature sensor or a thermography, or a device including a temperature sensor, a thermography, or the like. For example, the first acquisition device 11 may be an air conditioner including a temperature sensor. The first acquisition device 11 is, but not limited to, a device capable of acquiring at least data of a temperature around a sleeping person.

### (2-1-2) Second Acquisition Device 12

The second acquisition device 12 is a device such as a skin temperature sensor, or a device including a skin temperature sensor or the like. For example, the second acquisition device 12 may be a wearable sensor including a skin temperature sensor. The second acquisition device 12 is, but not limited to, a device capable of acquiring at least data of a skin temperature of the sleeping person.

### (2-1-3) Third Acquisition Device 13

The third acquisition device 13 is a device such as a core body temperature sensor, or a device including a core body temperature sensor or the like. For example, the third acquisition device 13 may be an eardrum thermometer. The third acquisition device 13 is, but not limited to, a device capable of acquiring at least data of a core body temperature of the sleeping person.

### (2-1-4) Fourth Acquisition Device 14

The fourth acquisition device 14 is a device for acquiring the quality of sleep of the sleeping person. In the first embodiment, the quality of sleep is determined based on a questionnaire on the quality of sleep or on the daytime performance for the sleeping person. The questionnaire on the quality of sleep or on the daytime performance is, for example, the OSA sleep inventory MA version. For example, the fourth acquisition device 14 may be a mobile terminal or the like. For example, the sleeping person performs the OSA sleep inventory MA version via the mobile terminal after waking up, and thus a mobile terminal that serves as the fourth acquisition device 14 acquires the data related to sleep. The fourth acquisition device 14 is not limited to a mobile terminal and may be any device capable of acquiring a result of the OSA sleep inventory MA version performed by the sleeping person.

### (2-2) Training Data Generation Apparatus 20

The training data generation apparatus 20 may be a computer which has a well-known hardware configuration such as a CPU (Central Processing Unit), a memory, a storage, an input/output interface, a communication interface, an input device, a display device, and a data bus, on which a well-known operating system or the like is installed, and which further has a function of a server. In addition, a GPU (Graphics Processing Unit) may be provided as required. Alternatively, the training data generation apparatus 20 may be a virtualized virtual server provided by using one or a plurality of computers. Note that the training data generation apparatus 20 in the present disclosure preferably includes a large-capacity memory.

The training data generation apparatus 20 includes a data acquisition unit 21, a feature quantity extraction unit 22, a training data generation unit 23, an acquired data storage unit 24, and a training data storage unit 25.

### (2-2-1) Data Acquisition Unit 21

The data acquisition unit 21 performs processing of acquiring various kinds of data acquired by the data acquisition apparatus 10 by receiving the data via the network. Each of the various kinds of data acquired by the data acquisition unit 21 is stored in the acquired data storage unit 24. Note that each of the various kinds of data stored in the acquired data storage unit 24 is associated with information of the time when the data is acquired by the data acquisition apparatus 10.

In the first embodiment, the data acquisition unit 21 acquires data of a first ambient temperature and a second ambient temperature from the first acquisition device 11, data of a first skin temperature and a second skin temperature from the second acquisition device 12, data of a first core body temperature and a second core body temperature from the third acquisition device 13, and data of the quality of sleep from the fourth acquisition device 14. Each piece of data will be described below. In description below, it is preferable to acquire the sleep onset time of the sleeping person at a timing when the sleeping person falls asleep by measuring the sleep onset of the sleeping person by using a sensor or the like. However, the configuration is not limited to this. The sleep onset time of the sleeping person may be a timing at least after the sleeping person lies down, for example, a timing after a predetermined time passes since the sleeping person lies down. The sleep onset time of the sleeping person in the first embodiment is after 30 minutes passes since the sleeping person lies down. However, the sleep onset time is not limited to this.

### (2-2-1-1) Data of First Ambient Temperature and Second Ambient Temperature

The first ambient temperature is a temperature around the sleeping person at sleep onset of the sleeping person. The second ambient temperature is a temperature around the sleeping person acquired at a timing before that of the first ambient temperature. Data of the second ambient temperature is acquired at a timing at least before the sleeping person lies down. For example, the data of the second ambient temperature may be data acquired any length of time before the sleeping person lies down. In the first embodiment, the data of the second ambient temperature is data of the temperature around the sleeping person 30 minutes before the sleeping person lies down. However, the data of the second ambient temperature is not limited to this.

### (2-2-1-2) Data of First Skin Temperature and Second Skin Temperature

The first skin temperature is a skin temperature at sleep onset of the sleeping person.

The second skin temperature is a skin temperature of the sleeping person acquired at a timing before that of the first skin temperature. It is preferable to acquire data of the second skin temperature at a timing before the sleeping person lies down. For example, the data of the second skin temperature is preferably data acquired any length of time before the sleeping person lies down. In the first embodiment, the data of the second skin temperature is data of the skin temperature of the sleeping person 30 minutes before the sleeping person lies down. However, the data of the second skin temperature is not limited to this.

### (2-2-1-3) Data of First Core Body Temperature and Second Core Body Temperature

The first core body temperature is a core body temperature at sleep onset of the sleeping person. The second core body temperature is a core body temperature of the sleeping person acquired at a timing before that of the first core body temperature. It is preferable to acquire data of the second core body temperature at a timing before the sleeping person lies down. For example, the data of the second core body temperature is preferably data acquired any length of time before the sleeping person lies down. In the first embodiment, the data of the second core body temperature is data of the core body temperature of the sleeping person 30 minutes before the sleeping person lies down. However, the data of the second core body temperature is not limited to this.

### (2-2-1-4) Data of Quality of Sleep

As described above, the quality of sleep is determined based on the OSA sleep inventory MA version. The data of the quality of sleep is acquired at a timing after the OSA sleep inventory MA version is demonstrated after the sleeping person wakes up.

### (2-2-2) Feature Quantity Extraction Unit 22

The feature quantity extraction unit 22 performs processing of extracting a skin temperature feature quantity and a core body temperature feature quantity respectively from the data of the first and second skin temperatures and the data of the first and second core body temperatures stored in the acquired data storage unit 24. The skin temperature feature quantity and the core body temperature feature quantity extracted by the feature quantity extraction unit 22 are stored in the acquired data storage unit 24. The skin temperature feature quantity and the core body temperature feature quantity will be described below.

### (2-2-2-1) Skin Temperature Feature Quantity

The skin temperature feature quantity is a feature quantity related to the skin temperature of the sleeping person and is extracted from the data of the first skin temperature and the second skin temperature. Specifically, the skin temperature feature quantity is a feature quantity determined based on any of a value of change in, a largest value of, a smallest value of, a mode value of, an average value of, a difference between the largest value and the smallest value of, a largest or smallest slope of, an average slope of, and an index related to the change in the first skin temperature and the second skin temperature. The index related to the change is an index indicating a tendency of the change such as, for example, a slope of the core body temperature that correlates to the skin temperature. A technique of extracting the skin temperature feature quantity from the data of the first skin temperature and the second skin temperature is not limited but it is preferable to select a useful feature quantity by selecting a feature using a filter method, a wrapper method, an embedded method, or the like. An interaction feature quantity may also be used.

### (2-2-2-2) Core Body Temperature Feature Quantity

The core body temperature feature quantity is a feature quantity related to the core body temperature of the sleeping person and is extracted from the data of the first core body temperature and the second core body temperature. Specifically, the core body temperature feature quantity is a feature quantity determined based on any of a value of change in, a largest value of, a smallest value of, a mode value of, an average value of, a difference between the largest value and the smallest value of, a largest or smallest slope of, an average slope of, and an index related to the change in the first core body temperature and the second core body temperature. The index related to the change is an index indicating a tendency of the change such as, for example, a slope of the skin temperature that correlates to the core body temperature. A technique of extracting the core body temperature feature quantity from the data of the first core body temperature and the second core body temperature is not limited but it is preferable to select a useful feature quantity by selecting a feature using a filter method, a wrapper method, an embedded method, or the like. An interaction feature quantity may also be used.

### (2-2-3) Training Data Generation Unit 23

The training data generation unit 23 extracts a state variable and ground truth data from the acquired data storage unit 24 and generates training data (training dataset). The training data is a dataset with which the learning apparatus 30 learns the quality of sleep. In the first embodiment, the data of the first ambient temperature, the second ambient temperature, the skin temperature feature quantity, the core body temperature feature quantity, and the quality of sleep is used as the training data.

Specifically, the training data generation unit 23 first extracts the data of the first ambient temperature, the second ambient temperature, the skin temperature feature quantity, the core body temperature feature quantity, and the quality of sleep from the acquired data storage unit 24. At this time, the training data generation unit 23 extracts each of the pieces of data, based on the information of the time associated with the piece of data. The training data generation unit 23 then generates training data by using, as the state variables, the first ambient temperature, the second ambient temperature, the skin temperature feature quantity, and the core body temperature feature quantity and by using, as the ground truth data, the data of the quality of sleep. The training data generated by the training data generation unit 23 is stored in the training data storage unit 35.

### (2-3) Learning Apparatus 30

The learning apparatus 30 is a high-performance computer which has a well-known hardware configuration such as a CPU (Central Processing Unit), a memory, a storage, an input/output interface, a communication interface, an input device, a display device, and a data bus that are not illustrated, on which a well-known operating system or the like is installed, and which further has a function of a server. In addition, a GPU (Graphics Processing Unit) may be provided as required. Alternatively, the learning apparatus 30 may be a virtualized virtual server provided by using one or a plurality of computers.

The learning apparatus 30 includes a training data acquisition unit 31, a learning unit 32, and a learning model generation unit 33.

### (2-3-1) Training Data Acquisition Unit 31

The training data acquisition unit 31 acquires a plurality of pieces of training data from the training data generation apparatus 20 via the network. Each of the pieces of training data acquired by the training data acquisition unit 31 is stored in a memory or the like.

### (2-3-2) Learning Unit 32

A learning model is provided in advance in the learning unit 32. The learning unit 32 causes the learning model to perform learning (for example, supervised learning) based on the plurality of pieces of training data acquired by the training data acquisition unit 31. A technique of learning performed by a learning model is not limited but it is preferable to use multiple regression analysis, random forest, or the like.

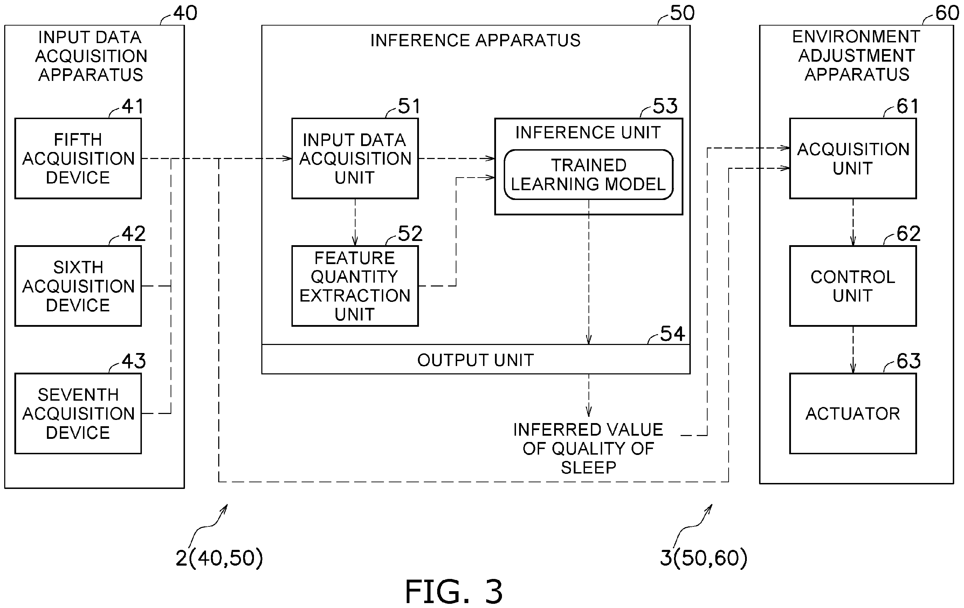

The learning model in the first embodiment performs learning for the purpose of outputting an inferred value of the quality of sleep from input data input to the inference system 2 (Fig. 3) described later.

### (2-3-3) Learning Model Generation Unit 33

The learning model generation unit 33 generates a learning model to be used by an inference apparatus 50, by outputting, as a trained learning model, the learning model at a stage where learning has progressed in the learning unit 32 and the inference accuracy of the learning model has improved. The learning model output from the learning model generation unit 33 is capable of outputting, with a predetermined accuracy or higher, the inferred value of the quality of sleep from input data input to the inference system 2.

The learning model generated by the learning model generation unit 33 is distributed to the inference apparatus 50 described later via the network. Alternatively, the learning model generated by the learning model generation unit 33 is distributed to the inference apparatus 50 via a storage medium.

### (3) Features of Learning System 1

### (3-1)

The learning apparatus 30 included in the learning system 1 of the first embodiment is the learning apparatus 30 that learns the quality of sleep of a sleeping person. The learning apparatus 30 includes the training data acquisition unit 31 that serves as an acquisition unit, the learning unit 32, and the learning model generation unit 33 that serves as a generation unit. The training data acquisition unit 31 acquires, as state variables, a first ambient temperature, a second ambient temperature, a feature quantity related to a core body temperature, and a feature quantity related to a skin temperature. The first ambient temperature is a temperature around the sleeping person at sleep onset of the sleeping person. The second ambient temperature is a temperature around the sleeping person acquired before the first ambient temperature. The feature quantity related to the core body temperature is determined based on at least a first core body temperature at the sleep onset of the sleeping person. The feature quantity related to the skin temperature is determined based on at least a first skin temperature at the sleep onset of the sleeping person. The learning unit 32 learns the state variables and the quality of sleep in association with each other. The learning model generation unit 33 generates a learning model based on a learning result of the learning unit 32. The learning model receives, as inputs, the first ambient temperature, the second ambient temperature, the feature quantity related to the core body temperature, and the feature quantity related to the skin temperature, and infers the quality of sleep.

Studies on the effects of changes in pre-bedtime body temperature on sleep onset have been hitherto conducted as in NPL 1. For example, NPL 1 reports that a great sleepiness is induced in a time period in which the core body temperature lowers, in particular, the appearance time of an abrupt decrease in core body temperature greatly affects the quality of sleep.

Specifically, when a person gets sleepy, heat of the core body temperature moves to the peripheral portion to dilate blood vessels for body temperature adjustment called AVA (arteriovenous anastomosis) present at the palms, the soles, and the face and to dissipate the heat. Consequently, the core body temperature lowers, and the resting efficiency of the brain and body during sleep can be increased. The core body temperature is likely to decrease at the bedtime if the body temperature is raised by doing exercise in the daytime or by taking a bath. However, if the core body temperature does not rise in the daytime or it is hot in a bedroom, the core body temperature does not lower. Consequently, a person cannot sleep deeply, resulting in a poor quality of sleep. It is characterized that deep sleep often appears in the first three hours from sleep onset and decreases thereafter. When the comfort during sleep is expressed as a deep and good quality of sleep, the deep and good quality of sleep is affected by a transition of the core body temperature. In summary, a relationship among the core body temperature, the skin temperature, and the sleep depth in ideal sleep is as illustrated in Fig. 5. In addition, because of aging, a sleep induction time increases, a proportion of deep sleep decreases, and a total sleep time decreases.

The learning apparatus 30 of the first embodiment acquires, as the state variable, the feature quantity related to the core body temperature of the sleeping person and performs learning so as to be able to generate a learning model capable of more accurately inferring the quality of sleep that is the sleep state of the sleeping person.

### (3-2)

A second feature quantity acquired by the learning apparatus 30 is determined based on at least the first skin temperature and the second skin temperature. The first skin temperature is a skin temperature at sleep onset of the sleeping person. The second skin temperature is a skin temperature of the sleeping person acquired before the first skin temperature.

In the present embodiment, the learning apparatus 30 acquires, as the first skin temperature, data of the skin temperature of the sleeping person after an elapse of 30 minutes since the sleeping person lies down, and acquires, as the second skin temperature, data of the skin temperature of the sleeping person 30 minutes before the sleeping person lies down. The learning apparatus 30 of the present embodiment acquires the first core body temperature at the same time as the first skin temperature, and acquires the second core body temperature at the same time as the second skin temperature.

Thus, the learning apparatus 30 can generate a learning model that outputs a more accurate core body temperature feature quantity.

### (3-3)

The learning apparatus 30 performs learning by using a plurality of pieces of training data. The training data includes the data of the quality of sleep, the data of the first ambient temperature, the data of the second ambient temperature, the core body temperature feature quantity that serves as the feature quantity related to the core body temperature, and the skin temperature feature quantity that serves as the feature quantity related to the skin temperature.

In the present embodiment, the quality of sleep is determined based on at least any one of a period from lying down to sleep onset of the sleeping person, a proportion of deep sleep, the number of times of arousal during sleep, a duration of arousal during sleep, and the OSA sleep inventory MA version.

The core body temperature feature quantity is determined based on at least any one of an amount of change in, a largest value of, a smallest value of, a mode value of, an average value of, a difference between the largest value and the smallest value of, a largest or smallest slope of, and an average slope of the core body temperature of the sleeping person in a predetermined period.

The skin temperature feature quantity is determined based on at least any one of an amount of change in, a largest value of, a smallest value of, a mode value of, an average value of, a difference between the largest value and the smallest value of, a largest or smallest slope of, and an average slope of the skin temperature of the sleeping person in a predetermined period.

Thus, the learning apparatus 30 of the present embodiment can generate a learning model that outputs an accurate quality of sleep.

### (4) Modifications

### (4-1) First Modification

In the first embodiment, the quality of sleep is determined based on the OSA sleep inventory MA version. However, the quality of sleep may be determined based on a method other than this. In addition, the fourth acquisition device 14 may be a device other than a mobile terminal.

For example, the quality of sleep may be determined based on a heart rate, body movements, a proportion of a deep sleep time of the sleeping person during sleep, a period from lying down to sleep onset of the sleeping person, a hormone secretion of the sleeping person, a hormone concentration of the sleeping person, any questionnaire on the quality of sleep or the daytime performance for the sleeping person, or the like. The data of the quality of sleep is not limited. The fourth acquisition device 14 is not limited as long as the fourth acquisition device 14 is capable of acquiring at least the data of the quality of sleep.

### (4-2) Second Modification

The data acquisition apparatus 10 may acquire data other than the data mentioned above. For example, the data acquisition apparatus 10 may acquire environment data, target data, or sleep data. Specifically, the environment data is data of a humidity, an illuminance, a chromaticity, a scent, an air flow, or the like around the sleeping person. The target data is data of a fingertip blood flow rate of the sleeping person, an RGB image for use in inference of the fingertip blood flow rate of the sleeping person, a heart rate, a change in heart rate, a respiratory rate, a galvanic skin response, an amount of worn clothes/a type of bedding, a daytime activity state, a temperature of a pillow, or the like. The sleep data is data such as body movements, the sleeping state, or the time to sleep onset of the sleeping person. These pieces of data may be acquired by the first acquisition device 11, the second acquisition device 12, the third acquisition device 13, and the fourth acquisition device 14 included in the data acquisition apparatus 10 or a device other than these. The learning apparatus 30 can use, as the training data, the data acquired by those devices.

The learning apparatus 30 can generate a learning model that infers a more accurate core body temperature feature quantity by using the above-described data as the state variables.

### (4-3) Third Modification

In the first embodiment, the first ambient temperature and the second ambient temperature are acquired by the same device (the first acquisition device 11). However, the first ambient temperature and the second ambient temperature may be acquired by different devices.

The first acquisition device 11 or the like may acquire, as a third ambient temperature, data of a temperature around the sleeping person at a predetermined timing in addition to the first ambient temperature and the second ambient temperature.

### (4-4) Fourth Modification

In the first embodiment, the training data generation apparatus 20 extracts the skin temperature feature quantity and the core body temperature feature quantity. However, the learning apparatus 30 may extract the skin temperature feature quantity and the core body temperature feature quantity. Alternatively, the second acquisition device 12 may extract the skin temperature feature quantity, and the third acquisition device 13 may extract the core body temperature feature quantity. Processing of extracting a feature quantity from each piece of data may be performed by any device.

### (4-5) Fifth Modification

In the first embodiment, the learning system 1 extracts the skin temperature feature quantity and the core body temperature feature quantity and performs learning. However, the learning system 1 may perform learning by using deep learning. That is, the learning unit 32 may automatically extract an appropriate feature quantity from the skin temperature, the core body temperature, the first ambient temperature, and the second ambient temperature and perform learning. In this case, the skin temperature and the core body temperature may be acquired only at sleep onset of the sleeping person.

### (4-6) Sixth Modification

In the first embodiment, the training data is a training dataset related to the sleeping person. The plurality of pieces of training data used by the learning unit 32 in learning may be a training dataset related to different targets (sleeping persons).

On the other hand, the plurality of pieces of training data used by the learning unit 32 of the first embodiment in learning may be a training dataset related to a specific sleeping person. In this case, the data acquisition apparatus 10 may include a personal information acquisition device that acquires personal information of the specific sleeping person. The personal information acquisition device is, for example, a mobile terminal, and receives personal information of the specific sleeping person. The personal information is, for example, information of the normal skin temperature, the normal core body temperature, the age, the gender, the height, or the weight of the specific sleeping person, an average normal skin temperature of people of the same age, or the like.

Thus, the learning apparatus 30 can contribute to generation of a learning model that infers a more accurate core body temperature feature quantity for a specific sleeping person.

### (5) Inference System 2

Fig. 3 is a schematic diagram illustrating an example of the inference system 2 and the environment adjustment system 3 according to the first embodiment. The inference system 2 includes apparatuses such as an input data acquisition apparatus 40 and the inference apparatus 50. The individual apparatuses are capable of communicating with each other via a predetermined network such as Internet.

### (5-1) Input Data Acquisition Apparatus 40

The input data acquisition apparatus 40 includes a fifth acquisition device 41 that acquires data of a temperature around a sleeping person, a sixth acquisition device 42 that acquires data of a skin temperature of the sleeping person, and a seventh acquisition device 43 that acquires data of a core body temperature of the sleeping person. Each of the fifth acquisition device 41, the sixth acquisition device 42, and the seventh acquisition device 43 acquires predetermined data.

In the first embodiment, each of the fifth acquisition device 41, the sixth acquisition device 42, and the seventh acquisition device 43 continuously acquires the data from 30 minutes before the sleeping person lies down. However, the data may be acquired at predetermined time intervals or may be acquired at given timings. Each of the pieces of data acquired by the fifth acquisition device 41, the sixth acquisition device 42, and the seventh acquisition device 43 is associated with information of the acquisition time of the piece of data.

The pieces of data acquired by the fifth acquisition device 41, the sixth acquisition device 42, and the seventh acquisition device 43 are transmitted to the inference apparatus 50 via the network. Note that the fifth acquisition device 41, the sixth acquisition device 42, and the seventh acquisition device 43 may transmit, to the inference apparatus 50, only partial data corresponding to a request from the inference apparatus 50 among the acquired pieces of data.

The fifth acquisition device 41, the sixth acquisition device 42, and the seventh acquisition device 43 and the pieces of data acquired by the fifth acquisition device 41, the sixth acquisition device 42, and the seventh acquisition device 43 will be described below. In description below, it is preferable to acquire the sleep onset time of the sleeping person at a timing when the sleeping person falls asleep by measuring the sleep onset of the sleeping person by using a sensor or the like. However, the configuration is not limited to this. The sleep onset time of the sleeping person may be a timing at least after the sleeping person lies down, for example, a timing after any length of time passes since the sleeping person lies down. In the first embodiment, the data of the second ambient temperature is data of the temperature around the sleeping person 30 minutes before the sleeping person lies down. However, the data of the second ambient temperature is not limited to this.

### (5-1-1) Fifth Acquisition Device 41

The fifth acquisition device 41 is a device such as a temperature sensor or a thermography, or a device including a temperature sensor, a thermography, or the like. For example, the fifth acquisition device 41 may be an air conditioner including a temperature sensor, or the like. The fifth acquisition device 41 is, but not limited to, a device capable of acquiring at least data of a temperature around a sleeping person.

In the first embodiment, the fifth acquisition device 41 acquires at least a third ambient temperature and a fourth ambient temperature.

The third ambient temperature is data of an ambient temperature at sleep onset of the sleeping person. The fourth ambient temperature is data of a temperature around the sleeping person acquired before the third ambient temperature. The fourth ambient temperature is acquired at least before the sleeping person lies down. For example, as the fourth ambient temperature, data acquired any length of time before the sleeping person lies down may be used. In the first embodiment, data of the fourth ambient temperature is data of the temperature around the sleeping person 30 minutes before the sleeping person lies down. However, the data of the fourth ambient temperature is not limited to this.

### (5-1-2) Sixth Acquisition Device 42

The sixth acquisition device 42 is a device such as a skin temperature sensor, or a device including a skin temperature sensor or the like. For example, the sixth acquisition device 42 may be a wearable sensor or the like including a skin temperature sensor or the like. The sixth acquisition device 42 is, but not limited to, a device capable of acquiring at least data of a third skin temperature and a fourth skin temperature of the sleeping person.

The third skin temperature is a skin temperature at sleep onset of the sleeping person. The fourth skin temperature is a skin temperature of the sleeping person acquired before the third skin temperature. It is preferable to acquire data of the fourth skin temperature before the sleeping person lies down. For example, the data of the fourth skin temperature is preferably data acquired any length of time before the sleeping person lies down. In the first embodiment, the data of the fourth skin temperature is data of the skin temperature of the sleeping person 30 minutes before the sleeping person lies down. However, the data of the fourth skin temperature is not limited to this.

### (5-1-3) Seventh Acquisition Device 43

The seventh acquisition device 43 is a device such as a core body temperature sensor, or a device including a core body temperature sensor or the like. For example, the seventh acquisition device 43 may be an eardrum thermometer or the like. The seventh acquisition device 43 is, but not limited to, a device capable of acquiring at least data of a third core body temperature and a fourth core body temperature of the sleeping person.

The third core body temperature is a core body temperature at sleep onset of the sleeping person. The fourth core body temperature is a core body temperature of the sleeping person acquired at a timing before that of the third core body temperature. It is preferable to acquire data of the fourth core body temperature at a timing before the sleeping person lies down. For example, the data of the fourth core body temperature is preferably data acquired any length of time before the sleeping person lies down. In the first embodiment, the data of the fourth core body temperature is data of the core body temperature of the sleeping person 30 minutes before the sleeping person lies down. However, the data of the fourth core body temperature is not limited to this.

### (5-2) Inference Apparatus 50

The inference apparatus 50 is a high-performance computer which has a well-known hardware configuration such as a CPU (Central Processing Unit), a memory, a storage, an input/output interface, a communication interface, an input device, a display device, and a data bus, on which a well-known operating system or the like is installed, and which further has a function of a server. In addition, a GPU (Graphics Processing Unit) may be provided as required. Alternatively, the inference apparatus 50 may be a virtualized virtual server provided by using one or a plurality of computers.

A trained learning model generated in advance by the learning system 1 and various programs for executing this learning model are stored in a storage unit such as a memory of the inference apparatus 50.

The inference apparatus 50 includes an input data acquisition unit 51, a feature quantity extraction unit 52, an inference unit 53, and an output unit 54.

### (5-2-1) Input Data Acquisition Unit 51

The input data acquisition unit 51 acquires the data of the third ambient temperature and the fourth ambient temperature acquired by the fifth acquisition device 41, the data of the third skin temperature and the fourth skin temperature acquired by the sixth acquisition device 42, and the data of the third core body temperature and the fourth core body temperature acquired by the seventh acquisition device 43, by receiving the data via the network. Each of the pieces of data acquired by the input data acquisition unit 51 is stored in a memory or the like.

### (5-2-2) Feature Quantity Extraction Unit 52

The feature quantity extraction unit 52 extracts a skin temperature feature quantity from the data of the third skin temperature and the fourth skin temperature acquired by the input data acquisition unit 51. The feature quantity extraction unit 52 also extracts a core body temperature feature quantity from the data of the third core body temperature and the fourth core body temperature acquired by the input data acquisition unit 51. The skin temperature feature quantity and the core body temperature feature quantity are feature quantities that are substantially the same as the skin temperature feature quantity and the core body temperature feature quantity extracted by the feature quantity extraction unit 22 of the learning system. Thus, description is omitted.

### (5-2-3) Inference Unit 53

The inference unit 53 receives, as input data, the data of the third ambient temperature and the fourth ambient temperature acquired by the input data acquisition unit 51 and the skin temperature feature quantity and the core body temperature feature quantity extracted by the feature quantity extraction unit 22, and infers the quality of sleep. Inferring the quality of sleep is, for example, classifying the quality of sleep of the sleeping person into three steps (such as well, average, and poor). Inferring is, for example, supervised learning. The inference technique is not limited but it is preferable to use multiple regression analysis, random forest, or the like. For example, as a result of an inference experiment using these techniques, the correct answer rate for input data is 0.762, 0.930, or 0.803. Thus, the quality of sleep can be inferred with a high accuracy.

### (5-2-4) Output Unit 54

The output unit 54 outputs an inferred value of the quality of sleep inferred by the inference unit 53. Specifically, the output unit 54, as outputting, transmits the inferred value of the quality of sleep to another device or displays the inferred value on a display or the like.

### (6) Features of Inference System 2

The inference apparatus 50 included in the inference system 2 in the first embodiment infers the quality of sleep of a sleeping person by using a trained learning model generated based on a learning result of the learning apparatus 30.

Thus, the inference system 2 of the first embodiment can accurately infer the quality of sleep of the sleeping person by using the learning model generated by the learning system 1.

### (7) Modifications

### (7-1) First Modification

The input data acquisition unit 51 may acquire input data other than the data mentioned above. For example, the input data acquisition unit 51 may acquire environment data, target data, sleep data, or the like. The input data acquisition unit 51 acquires input data according to the specifications of the learning model.

### (7-2) Second Modification

In the first embodiment, the inference apparatus 50 extracts the skin temperature feature quantity. However, the sixth acquisition device 42 may extract the skin temperature feature quantity. Alternatively, another device other than the sixth acquisition device 42 may extract the skin temperature feature quantity.

### (7-3) Third Modification

In the first embodiment, the inference system 2 extracts the skin temperature feature quantity and performs learning. However, the inference system 2 may perform learning by using deep learning. That is, the inference unit 53 may automatically extract an appropriate feature quantity from the third ambient temperature, the fourth ambient temperature, the third skin temperature, and the fourth skin temperature and perform learning.

### (8) Environment Adjustment System 3

The environment adjustment system 3 is a system that adjusts an environment around a sleeping person, based on the quality of sleep of the sleeping person inferred by the inference system 2. The environment adjustment system 3 includes the inference apparatus 50 of the first embodiment, and an environment adjustment apparatus 60. An example of the environment adjustment apparatus 60 will be described below.

### (8-1) Environment Adjustment Apparatus 60

The environment adjustment apparatus 60 is an apparatus that adjusts an environment around a sleeping person and is, for example, an air conditioner. The environment adjustment apparatus 60 mainly includes an acquisition unit 61, a control unit 62, and an actuator 63.

### (8-1-1) Acquisition Unit 61

The acquisition unit 61 acquires an inferred value of the quality of sleep from the inference apparatus 50 via a network such as the Internet. The acquisition unit 61 also acquires various kinds of data from the input data acquisition apparatus 40 via the network. The acquisition unit 61 stores the information acquired from the inference apparatus 50 and the input data acquisition apparatus 40 in a storage device of the control unit 62 described later.

### (8-1-2) Control Unit 62

The control unit 62 is implemented by a computer and includes a control/arithmetic device and a storage device that are not illustrated. As the control/arithmetic device, a processor such as a CPU or a GPU can be used. The control/arithmetic device reads out a program stored in the storage device, and performs predetermined arithmetic processing in accordance with this program. The control/arithmetic device can further write the arithmetic operation result in the storage device or read out information stored in the storage device in accordance with the program.

Specifically, the storage device of the control unit 62 of the first embodiment stores the information acquired by the acquisition unit 61. The storage device also stores a control instruction given in a predetermined period before the acquisition unit 61 acquires the inferred value of the quality of sleep. The control instruction is an instruction signal for controlling an operation of the actuator 63 described later.

The control/arithmetic device of the control unit 62 performs an arithmetic operation based on the information and the control instruction stored in the storage device, and sends a control instruction for improving the quality of sleep to the actuator 63.

### (8-1-3) Actuator 63

The actuator 63 receives the control instruction from the control/arithmetic device of the control unit 62 and operates. The actuator 63 is, for example, a compressor, an expansion valve, a fan, a flap, or the like of an air conditioner.

### (9) Features

The environment adjustment system 3 in the first embodiment includes the inference apparatus 50 and the environment adjustment apparatus 60. The environment adjustment apparatus 60 includes the actuator 63 and the control unit 62. The actuator 63 adjusts an environment around a sleeping person. Based on the quality of sleep of the sleeping person inferred by the inference apparatus 50, the control unit 62 controls an operation of the actuator.

By using a human characteristic that a great sleepiness is induced when the core body temperature decreases as described in NPL 1, the environment adjustment system 3 decreases (for example, decreases by one degree) the temperature around the sleeping person so that the quality of sleep improves (the core body temperature decreases) in the case where the quality of sleep is predicted to decrease, for example. Alternatively, the environment adjustment system 3 adjusts the environment to increase the air flow around the sleeping person. Thus, the environment adjustment system 3 contributes to improvement of the quality of sleep of the sleeping person.

### (10) Modifications

In the first embodiment, the acquisition unit 61 acquires the various kinds of data from the input data acquisition apparatus 40. However, the acquisition unit 61 may acquire the various kinds of data from a sensor appropriately installed in the environment adjustment system 3.

The environment adjustment system 3 and the inference system 2 may be housed in the same housing. Thus, the acquisition unit 61 may acquire the pieces of information without via the network.

### <Second Embodiment

Details of a learning system 1 and an inference system 2 according to a second embodiment of the invention of this application will be described below in terms of differences from the respective systems of the first embodiment.

### (11) Learning System 1 of Second Embodiment

### (11-1) Data Acquisition Apparatus 10

A data acquisition apparatus 10 in the learning system 1 of the second embodiment is substantially the same as the data acquisition apparatus 10 of the first embodiment.

### (11-2) Training Data Generation Apparatus 20

In the learning system 1 of the second embodiment, a training data generation apparatus 20 generates training data used by a learning apparatus 30 to learn a core body temperature feature quantity of a sleeping person. For convenience, the training data generated for learning the quality of sleep of the sleeping person and described in the first embodiment is referred to as first training data, and training data for use in learning of the core body temperature feature quantity is referred to as second training data.

A training data generation unit 23 of the training data generation apparatus 20 first extracts a data of a first ambient temperature, a second ambient temperature, a skin temperature feature quantity, and a core body temperature feature quantity from an acquired data storage unit 24. The training data generation unit 23 then generates the second training data by using, as state variables, the first ambient temperature, the second ambient temperature, and the skin temperature feature quantity and by using, as ground truth data, the data of the core body temperature feature quantity. The second training data generated by the training data generation unit 23 is stored in a training data storage unit 35.

Note that the training data generation unit 23 of the second embodiment also generates the first training data as in the first embodiment.

### (11-3) Learning Apparatus 30

The learning apparatus 30 causes a learning model to perform learning based on the plurality of pieces of training data acquired by a training data acquisition unit 31. For convenience, a learning mode that learns the quality of sleep of a sleeping person by using the first training data is referred to as a first learning model, and a learning model that learns the core body temperature feature quantity by using the second training data is referred to as a second learning model. Note that the first learning model is the learning model described in the first embodiment.

By using the plurality of pieces of second training data acquired by the training data generation apparatus 20, the learning apparatus 30 performs learning for the purpose of outputting an inferred value of the core body temperature feature quantity from input data input to the inference system 2.

A learning model generation unit 33 generates the second learning model to be used by an inference apparatus 50, by outputting, as the trained second learning model, the second learning model at a stage where learning has progressed in a learning unit 32 and the inference accuracy of the learning model has improved. The second learning model output from the learning model generation unit 33 is capable of outputting, with a predetermined accuracy or higher, the inferred value of the core body temperature feature quantity from input data input to the inference system 2.

Note that the learning apparatus 30 of the second embodiment also generates, as the first learning model, the learning model that learns the quality of sleep of the sleeping person by using the first training data as in the first embodiment.

### (12) Features of Learning System 1 of Second Embodiment

The learning apparatus 30 included in the learning system 1 of the second embodiment is the learning apparatus 30 that learns the quality of sleep of a sleeping person. The learning apparatus 30 includes the training data acquisition unit 31 that serves as an acquisition unit, the learning unit 32, and the learning model generation unit 33 that serves as a generation unit. The training data acquisition unit 31 acquires, as state variables, a first ambient temperature, a second ambient temperature, a feature quantity related to a core body temperature, and a feature quantity related to a skin temperature. The first ambient temperature is a temperature around the sleeping person at sleep onset of the sleeping person. The second ambient temperature is a temperature around the sleeping person acquired before the first ambient temperature. The feature quantity related to the core body temperature is determined based on at least a first core body temperature at the sleep onset of the sleeping person. The feature quantity related to the skin temperature is determined based on at least a first skin temperature at the sleep onset of the sleeping person. The learning unit 32 learns the state variables and the quality of sleep in association with each other. The learning model generation unit 33 generates a learning model based on a learning result of the learning unit 32. The learning model receives, as inputs, the first ambient temperature, the second ambient temperature, and the feature quantity related to the skin temperature, and infers the quality of sleep.

The core body temperature of the sleeping person greatly affects the quality of sleep. However, to measure the core body temperature, a device such as a rectal thermometer or an eardrum thermometer needs to be attached. Such a device imposes a greater load on the sleeping person than in the case where the skin temperature is measured.

The learning apparatus 30 described in the second embodiment in advance acquires and learns information for use in calculation of the feature quantity related to the core body temperature of the sleeping person, and thus generates a learning model that outputs a first feature quantity that is information related to the core body temperature. Thus, a load imposed on the sleeping person by acquisition of the information related to the core body temperature can be reduced.

### (13) Modifications

In the second embodiment, the first training data and the second training data are generated by the same training data generation apparatus 20. However, the configuration is not limited to this. The first training data and the second training data may be generated by different pieces of hardware.

The first learning model and the second learning model are trained by the same learning apparatus 30. However, the configuration is not limited to this. The first learning model and the second learning model may be trained by different pieces of hardware.

### (14) Inference System 2

### (14-1) Input Data Acquisition Apparatus 40

An input data acquisition apparatus 40 of the second embodiment includes the fifth acquisition device 41 and the sixth acquisition device 42 of the first embodiment. That is, the input data acquisition apparatus 40 of the second embodiment does not include the seventh acquisition device 43. The fifth acquisition device 41 and the sixth acquisition device 42 acquire substantially the same pieces of data as those of the first embodiment.

### (14-2) Inference Apparatus 50

The inference apparatus 50 of the second embodiment stores the trained first learning model and the trained second learning model generated in advance by the learning system 1 and various programs for executing the individual learning models.

An inference unit 53 receives, as input data, the data of the third ambient temperature and the fourth ambient temperature acquired by an input data acquisition unit 51 and the skin temperature feature quantity extracted by a feature quantity extraction unit 22, and infers the core body temperature feature quantity by using the second learning model. The inference unit 53 can infer, as the core body temperature feature quantity, which of an increase by 0.2°C or more, flat (between a decrease by 0.2°C and an increase by 0.2°C), or a decrease by 0.2°C or more the change becomes when an average eardrum temperature per minute of a period from 30 to 16 minutes before the sleep onset and an average eardrum temperature per minute of a period from 15 minutes before to the sleep onset are compared with each other, for example. Inferring is, for example, supervised learning. In the case where the number of samples varies, a technique such as undersampling or oversampling can be employed. Table (1) below shows correct answer rates in experiments of inferring the core body temperature feature quantity by using the inference unit 53. In this manner, combinations of the experiment conditions for inferring the core body temperature feature quantity are compared with one another.

**[Table 1]**

| Experiment No. | Correct answer rate |
|---|---|
| 1 | 0.687 |
| 2 | 0.826 |
| 3 | 0.754 |
| 4 | 0.928 |
| 5 | 0.939 |
| 6 | 0.880 |
| ... | ... |

The inference apparatus 50 of the second embodiment infers the quality of sleep by using the core body temperature feature quantity inferred by the inference unit 53. Specifically, the inference unit 53 receives, as input data, the data of the third ambient temperature and the fourth ambient temperature acquired by the input data acquisition unit 51, the skin temperature feature quantity extracted by the feature quantity extraction unit 22, and the core body temperature feature quantity inferred by the inference unit 53, and infers the quality of sleep by using the first learning model. An output unit 54 outputs the inferred value of the quality of sleep inferred by using the first learning model.

### (15) Features

The inference apparatus 50 included in the inference system 2 in the second embodiment infers the quality of sleep of a sleeping person by using a trained learning model generated based on a learning result of the learning apparatus 30.

The inference system 2 further includes the inference unit 53 that serves as a core body temperature feature quantity inference unit. The inference unit 53 infers the feature quantity related to the core body temperature of the sleeping person from the first ambient temperature, the second ambient temperature, and the feature quantity related to the skin temperature.

Thus, the inference apparatus 50 can accurately infer the quality of sleep of the sleeping person and can also reduce the load imposed on the sleeping person by attachment of a device such as a rectal thermometer or an eardrum thermometer to acquire the information related to the core body temperature.

### (16) Modifications

In the second embodiment, the core body temperature feature quantity and the quality of sleep are inferred by the same inference apparatus 50. However, the configuration is not limited to this. The core body temperature feature quantity and the quality of sleep may be inferred by different pieces of hardware.

While the embodiments of the present disclosure have been described above, it should be understood that various modifications can be made on the configurations and details without departing from the gist and the scope of the present disclosure that are described in the claims.

### Reference Signs List

- 3: environment adjustment system
- 30: learning apparatus
- 31: acquisition unit
- 32: learning unit
- 50: inference apparatus
- 53: core body temperature feature quantity inference unit
- 62: control unit
- 63: actuator

### Citation List

### Non Patent Literature

NPL 1: <Paper> The effects of Pre-Bedtime changes in Body Temperature on Sleep Onset (General), ODA Shiro, http://id.nii.ac.jp/1136/00002386/

## Claims

1. A learning apparatus (30) that learns a quality of sleep of a sleeping person, comprising:
an acquisition unit (31) that acquires, as a state variable, a feature quantity related to a core body temperature of the sleeping person or a feature quantity related to a skin temperature of the sleeping person;
a learning unit (32) that learns the state variable and the quality of sleep in association with each other; and
a generation unit that generates, based on a learning result of the learning unit (32), a learning model that receives, as an input, the feature quantity related to the core body temperature or the feature quantity related to the skin temperature and infers the quality of sleep of the sleeping person, wherein
the feature quantity related to the core body temperature is determined based on at least a first core body temperature at sleep onset of the sleeping person, and
the feature quantity related to the skin temperature is determined based on at least a first skin temperature at the sleep onset of the sleeping person.

2. The learning apparatus (30) according to Claim 1, wherein
the feature quantity related to the core body temperature is determined based further on a second core body temperature acquired before the first core body temperature, and
the feature quantity related to the skin temperature is determined based further on a second skin temperature acquired before the first skin temperature.

3. The learning apparatus (30) according to Claim 1 or 2, wherein
the acquisition unit (31) further acquires a feature quantity related to a normal core body temperature of the sleeping person or a feature quantity related to a normal skin temperature of the sleeping person.

4. The learning apparatus (30) according to any of Claims 1 to 3, wherein
the acquisition unit (31) acquires, as the state variable, an ambient temperature of the sleeping person at the sleep onset, a fingertip blood flow rate of the sleeping person, or an RGB image of the sleeping person.

5. The learning apparatus (30) according to any of Claims 1 to 4, wherein
the quality of sleep is determined based on at least any one of a period from lying down to the sleep onset of the sleeping person, a proportion of deep sleep of the sleeping person, the number of times of arousal during sleep of the sleeping person, a duration of arousal during sleep of the sleeping person, a questionnaire on the quality of sleep for the sleeping person, a questionnaire on daytime performance for the sleeping person, a hormone secretion of the sleeping person, and a hormone concentration of the sleeping person.

6. The learning apparatus (30) according to any of Claims 1 to 5, wherein
the feature quantity related to the core body temperature is determined based on at least any one of an amount of change in, a largest value of, a smallest value of, a mode value of, an average value of, a difference between the largest value and the smallest value of, a largest or smallest slope of, an average slope of, and an index related to the change in the core body temperature of the sleeping person in a predetermined period.

7. The learning apparatus (30) according to any of Claims 1 to 6, wherein
the feature quantity related to the skin temperature is determined based on at least any one of an amount of change in, a largest value of, a smallest value of, a mode value of, an average value of, a difference between the largest value and the smallest value of, a largest or smallest slope of, an average slope of, and an index related to the change in the skin temperature of the sleeping person in a predetermined period.

8. The learning apparatus (30) according to any of Claims 1 to 7, wherein
the acquisition unit (31) further acquires, as the state variable, any of a humidity, an illuminance, a chromaticity, a scent, and an air flow around the sleeping person at the sleep onset of the sleeping person.

9. The learning apparatus (30) according to any of Claims 1 to 8, wherein
the learning unit (32) performs learning by using a plurality of pieces of training data, and
the training data includes the state variable and the quality of sleep.

10. An inference apparatus (50) that infers the quality of sleep of the sleeping person by using the learning model generated based on a learning result of the learning apparatus according to any of Claims 1 to 9.

11. The inference apparatus (50) according to Claim 10, further comprising:
a core body temperature feature quantity inference unit (53), wherein
the core body temperature feature quantity inference unit infers the feature quantity related to the core body temperature of the sleeping person from the feature quantity related to the skin temperature of the sleeping person.

12. The inference apparatus (50) according to Claim 10, wherein the core body temperature feature quantity inference unit (53)
infers the feature quantity related to the core body temperature of the sleeping person by receiving, as an input, the feature quantity related to the skin temperature of the sleeping person, based on a learning result of the learning model that has learned the feature quantity related to the core body temperature of the sleeping person in association with the feature quantity related to the skin temperature of the sleeping person,
the feature quantity related to the skin temperature of the sleeping person is determined based on at least the first skin temperature at sleep onset of the sleeping person, and
the feature quantity related to the core body temperature of the sleeping person is determined based on at least the first core body temperature at the sleep onset of the sleeping person.

13. An environment adjustment system (3) comprising:
the inference apparatus (50) according to any of Claims 10 to 12; and
an environment adjustment apparatus (60), wherein
the environment adjustment apparatus comprises an actuator (63) that adjusts an environment around a sleeping person, and a control unit (61) that controls an operation of the actuator (63), and
the control unit (61) controls the operation of the actuator (63), based on the quality of sleep of the sleeping person inferred by the inference apparatus (50).
